# EUROPEAN PATENT SPECIFICATION

(11) **EP 4 292 639 B1**
(45) Date of publication and mention of the grant of the patent: **01.04.2026**
(21) Application number: 22862959.8
(22) Date of filing: 26.07.2022
(51) Int. Cl.: A61M 60/178, A61M 60/216, A61M 60/863

(54) **VENTRICULAR CONNECTING ASSEMBLY AND VENTRICULAR ASSIST SYSTEM**
VENTRIKULÄRE VERBINDUNGSANORDNUNG UND VENTRIKULÄRES UNTERSTÜTZUNGSSYSTEM
ENSEMBLE DE CONNEXION VENTRICULAIRE ET SYSTÈME D'ASSISTANCE VENTRICULAIRE

(30) Priority: 06.09.2021 CN 202111037283
(43) Date of publication of application: 20.12.2023
(73) Proprietor: Shenzhen Core Medical Technology Co., Ltd., Shenzhen, Guangdong 518051 (CN)
(72) Inventor: XIE, Duanqing, Shenzhen, Guangdong 518000 (CN); YU, Shunzhou, Shenzhen, Guangdong 518000 (CN)
(74) Representative: Appleyard Lees IP LLP
(86) International application number: PCT/CN2022/107971
(87) International publication number: WO 2023/029818

(56) References cited:
- EP-A2- 2 213 192
- WO-A1-2014/149892
- CN-A- 106 794 294
- CN-A- 107 961 407
- CN-A- 113 908 428
- CN-U- 211 834 545
- CN-U- 211 834 545
- CN-U- 213 139 118
- US-A1- 2011 118 833
- US-A1- 2013 178 694

## Description

### TECHNICAL FIELD

The present application relates to the technical field of medical devices, in particular to a ventricular connecting assembly and a ventricular assist system.

### BACKGROUND

In blood pump implantation operations, it is usually necessary to use a ventricular connecting assembly to fix a blood pump on a biological tissue (see e.g. US 2011/118833 A1 and CN 211834545 U). However, the current ventricular connecting assembly is cumbersome to operate, which will bring a lot of inconvenience to the mounting of the blood pump during use.

### SUMMARY

### Technical problem

The present application provides a ventricular connecting assembly and a ventricular assist system to solve the problem that the operation of the ventricular connecting assembly is cumbersome and brings a lot of inconvenience to the mounting of the blood pump during use.

### Technical solution

The invention is defined by the features of the independent claims. The dependent claims concern particular embodiments.
In a first aspect, the present application provides a ventricular connecting assembly, including:
a clamping assembly including a split ring, a first connecting arm, and a second connecting arm, the split ring including a first end and a second end spaced apart from the first end, an inner diameter of the split ring being adjustable, so that the split ring has a clamping state and a non-clamping state, and the first connecting arm and the second connecting arm being connected to the first end and the second end of the split ring, respectively; and
a locking assembly including a connecting member and a locking member, the connecting member being connected to the second connecting arm, the locking member being rotatably connected to the connecting member, the locking member being capable of rotatably abutting against the first connecting arm, wherein the locking member is capable of adjusting relative positions of the first connecting arm and the second connecting arm when the locking member rotates, so as to adjust the inner diameter of the split ring, and the locking member is engaged with the second connecting arm to maintain the split ring in the clamped state.

In a second aspect, the present application further provides a ventricular assist system, including a blood pump including an inlet tube; and
the above-mentioned ventricular connecting assembly, wherein the inlet tube extends through the split ring, and the split ring is capable of clamping the inlet tube when the split ring is in the clamping state.

In a third aspect, the present application also provides a ventricular assist system, including a blood pump and a ventricular connecting assembly. The blood pump includes an inlet tube, the ventricular connecting assembly includes:
a sewing ring;
a clamping assembly including a split ring, a first connecting arm, and a second connecting arm, the split ring including a first end and a second end spaced apart from the first end, an inner diameter of the split ring being adjustable, so that the split ring has a clamping state and a non-clamping state, and the first connecting arm and the second connecting arm being connected to the first end and the second end of the split ring, respectively, the annular disk being annular with an opening, and the annular disk being connected with the sewing ring, wherein the split ring is provided in an inner ring of the annular disk, a position of the opening of the split ring corresponds to a position of the opening of the annular disk, an outer ring surface of the split ring adjacent to the second end is fixedly connected to the inner ring surface of the annular disk, and an outer ring surface of the split ring adjacent to the first end is separated from the annular disk;
a locking assembly including a connecting member and a locking member, the connecting member being connected to the second connecting arm, the locking member being rotatably connected to the connecting member, and the locking member being capable of rotatably abutting against the first connecting arm, wherein the locking member is capable of adjusting relative positions of the first connecting arm and the second connecting arm when the locking member rotates, so as to adjust the inner diameter of the split ring, and the locking member is engaged with the second connecting arm to maintain the split ring in the clamped state;
wherein the inlet tube extends through the split ring, and the split ring is capable of clamping the inlet tube when the split ring is in the clamping state.

### BRIEF DESCRIPTION OF THE DRAWINGS

In order to illustrate the embodiments of the present application more clearly, the drawings used in the embodiments will be described briefly. Apparently, the following described drawings are merely for the embodiments of the present application, and other drawings can be derived by those of ordinary skill in the art without any creative effort.
FIG. 1 is a schematic view of a ventricular connecting assembly according to an embodiment of the present application.
FIG. 2 is a schematic view of the ventricular connecting assembly shown in FIG. 1 viewed from another aspect.
FIG. 3 is an exploded view of the ventricular connecting assembly shown in FIG. 1.
FIG. 4 is a schematic view of a clamping assembly of the ventricular connecting assembly shown in FIG. 1.
FIG. 5 is a schematic view of the clamping assembly shown in FIG. 4 viewed from another aspect.
FIG. 6 is a schematic view of the ventricular connecting assembly shown in FIG. 1 viewed from another aspect.
FIG. 7 is a schematic view of a locking member of the ventricular connecting assembly shown in FIG. 2.
FIG. 8 is a schematic view of a blood pump according to the present application.

### DETAILED DESCRIPTION OF THE EMBODIMENTS

The present application will now be described in detail with reference to the accompanying drawings and embodiments in order to make the objects, technical solutions, and advantages of the present application clearer. It should be understood that the specific embodiments described herein are only for explaining the present application, and not intended to limit the present application.

It should be noted that when an element is called "fixed to" or "provided on" another element, it can be directly on another element or there can be a centered element. When an element is considered to be "connected" to another element, it can be directly connected to another element or indirectly connected to the other element.

In addition, the terms "first" and "second" are only used for descriptive purposes, and should not be construed as indicating or implying relative importance or implying the number of indicated technical features. Thus, a feature delimited with "first", "second" may expressly or implicitly include at least one of the features. In the description of the present application, "plurality" means two or more, unless otherwise expressly and specifically defined.

In order to illustrate the technical solution of the present application, the specific drawings and embodiments will be combined to illustrate below.

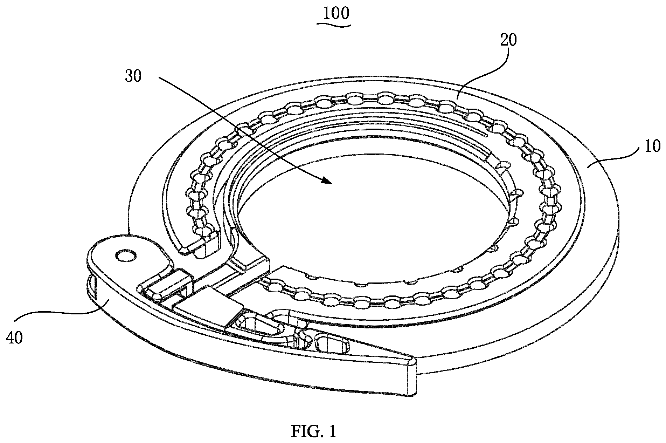

As shown in FIG. 1, an embodiment of a first aspect of the present application provides a ventricular connecting assembly 100, which especially relates to a ventricular connecting assembly 100 capable of connecting a blood pump to a biological tissue (e.g., heart tissue). The ventricular connecting assembly 100 includes a sewing ring 10, a clamping assembly 20, and a locking assembly 40.

The sewing ring 10 is configured to be secured to the biological tissue. The sewing ring 10 is of an annular structure. In some embodiments, the sewing ring 10 is sutured to the biological tissue by sutures. In some embodiments, the sewing ring 10 includes an annular cushion and a fabric layer wrapping the annular cushion. For example, the annular cushion is made of implantable silicone, and the fabric layer is made of polyester cloth.

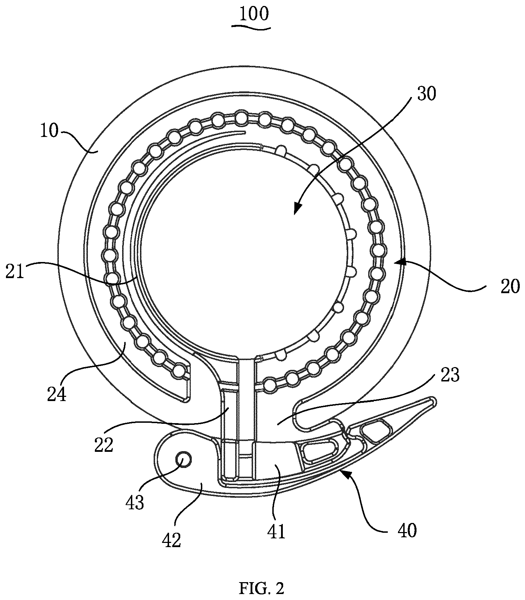

The clamping assembly 20 is connected to the sewing ring 10. In some embodiments, the clamping assembly 20 is fixedly connected to the sewing ring 10 by sutures. In other embodiments, the clamping assembly 20 may also be connected to the sewing ring 10 by adhesives, etc. The clamping assembly 20 is configured to clamp the blood pump (e.g., an inlet tube of the blood pump). The clamping assembly 20 is provided with a clamping opening 30, the inlet tube of the blood pump can extend through the clamping opening 30 and be clamped in clamping opening 30. Referring to FIG. 2, specifically, the clamping assembly 20 includes a split ring 21, a first connecting arm 22, a second connecting arm 23, and an annular disk 24.

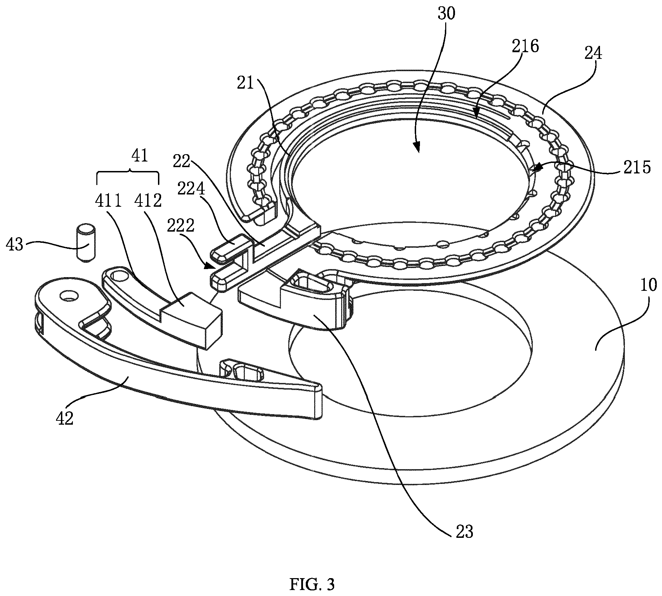

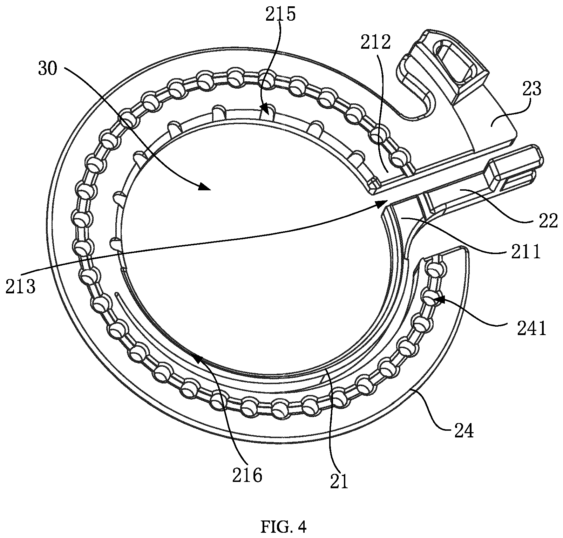

Referring to FIG. 3 and FIG. 4, the split ring 21 is configured to clamp the blood pump. The split ring 21 has a first end 211 and a second end 212 spaced apart from the first end 211. An inner diameter of the split ring 21 is adjustable, so that the split ring 21 has a clamping state and a non-clamping state. The clamping opening 30 is surrounded by the split ring 21. By adjusting the relative positions of the first end 211 and the second end 212, the inner diameter of the split ring 21 (that is, a size of the clamping opening 30) can be adjusted.

In the illustrated embodiment, the split ring 21 has a substantially annular structure with an opening 213. The first end 211 and the second end 212 of the split ring 21 are opposite and spaced apart to form the opening 213. By adjusting a distance between the first end 211 and the second end 212 (or a size of the opening 213), the inner diameter of the split ring 21 can be adjusted. When the inner diameter of the split ring 21 increases, the distance between the first end 211 and the second end 212 of the split ring 21 increases, and when the inner diameter of the split ring 21 decreases, the distance between the first end 211 and the second end 212 of the split ring 21 decreases.

The inner diameter of the split ring 21 in the clamped state is less than the inner diameter of the split ring 21 in the non-clamping state. That is, when the split ring 21 is in the clamping state, the split ring 21 has a smaller inner diameter. At this time, the split ring 21 can clamp the inlet tube of the blood pump. When the split ring 21 is in the non-clamping state, the split ring 21 has a larger inner diameter, the split ring 21 cannot clamp the inlet tube of the blood pump. At this time, the split ring 21 can loosen the inlet tube, or allow the inlet tube of the blood pump to extend through.

The first connecting arm 22 and the second connecting arm 23 are connected to the first end 211 and the second end 212 of the split ring 21, respectively. When the inner diameter of the split ring 21 increases, a distance between the first connecting arm 22 and the second connecting arm 23 increases. When the inner diameter of the split ring 21 decreases, the distance between the first connecting arm 22 and the second connecting arm 23 decreases. Specifically, the first connecting arm 22 and the second connecting arm 23 are bent and extended from the first end 211 and the second end 212 of the split ring 21, respectively, in a direction away from the split ring 21.

In one of the embodiments, in the non-clamping state (or in an initial state), a side of the first connecting arm 22 facing the second connecting arm 23 is parallel to a side of the second connecting arm 23 facing the first connecting arm 22. In other embodiments, in the non-clamping state (or in the initial state), from an end adjacent to the split ring 21 to an end away from the split ring 21, a distance between the side of the first connecting arm 22 facing the second connecting arm 23 and the side of the second connecting arm 23 facing the first connecting arm 22 gradually increases. It should be understood that, in other embodiments, in the non-clamping state, from the end adjacent to the split ring 21 to the end away from the split ring 21, a distance between the side of the first connecting arm 22 facing the second connecting arm 23 and the side of the second connecting arm 23 facing the first connecting arm 22 gradually decreases.

In some embodiments, the first connecting arm 22 and the first end 211 of the split ring 21 may be integrally formed. Alternatively, the independent first connecting arm 22 is connected to the first end 211 of the split ring 21 by, such as welding, etc. The second connecting arm 23 may be integrally formed with the second end 212 of the split ring 21. Alternatively, the independent second connecting arm 23 is be connected to the second end 212 of the split ring 21 by, such as welding, etc.

The annular disk 24 is fixedly connected to the sewing ring 10. In one embodiment, the annular disk 24 is fixedly connected to the sewing ring 10 by sutures. In the embodiment, the annular disk 24 is provided with a suture hole 241, and the sewing ring 10 and the annular disk 24 are fixedly connected by sutures extending through the sewing ring 10 and the suture hole 241. It should be understood that, in other embodiments, the annular disk 24 may also be connected to the sewing ring 10 by adhesive, etc.

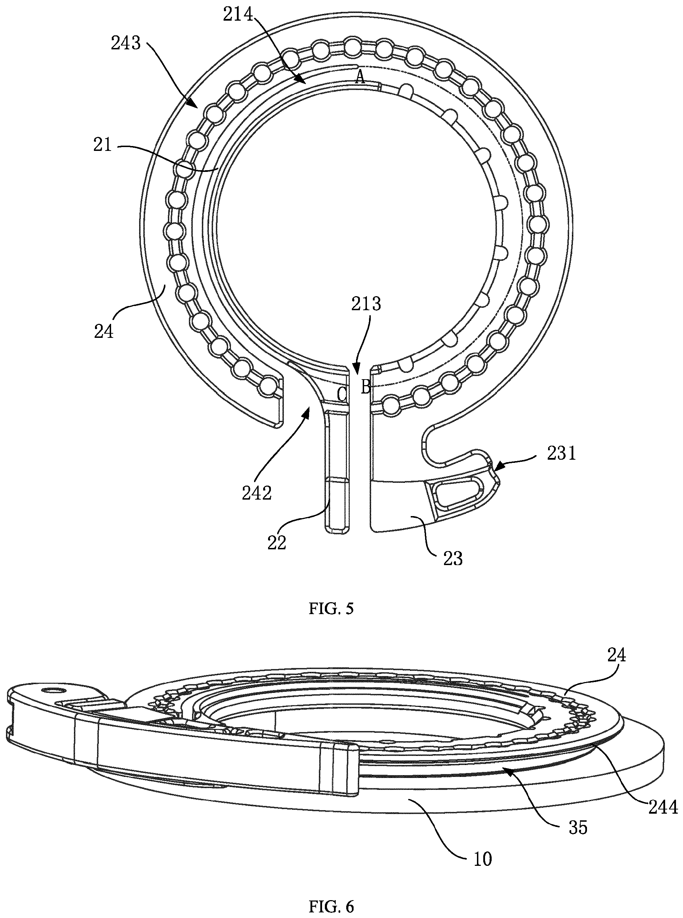

Referring to FIG. 5, the annular disk 24 has an annular structure with an opening 242. The split ring 21 is provided in an inner ring of the annular disk 24, a position of the opening 213 of the split ring corresponds to a position of the opening 242 of the annular disk 24. An outer ring surface of the split ring 21 adjacent to the second end 212 (i.e., a portion of the outer ring surface corresponding to the arc AB shown in the figure) is fixedly connected to the inner ring surface of the annular disk 24, and an outer ring surface of the split ring 21 adjacent to the first end 211 (i.e., a portion of the outer ring surface corresponding to the arc AC shown in the figure) is separated from the annular disk 21. That is, a part of the outer ring surface of the split ring 21 is fixedly connected to the inner ring surface of the annular disk 24, and another part of the outer ring surface of the split ring 21 is separated from the inner ring surface of the annular disk 24. The above-mentioned method can not only ensure the reliable connection between the split ring 21 and the annular disk 24, but also ensure that the split ring 21 has a certain degree of elasticity, which facilitates the adjustment of the inner diameter of the split ring 21.

In one of the embodiments, a length of the portion of the outer ring surface of the split ring 21 fixedly connected to the inner ring face of the annular disk 24 (approximately a length of the arc AB shown in the figure) is less than or equal to half of a length of the outer ring face of the split ring 21 (approximately a length of the arc BC shown in the figure). That is, along a circumferential direction of the split ring 21, the length of the portion of the outer ring surface of the split ring 21 fixedly connected to the inner ring face of the annular disk 24 is less than or equal to half of the length of the outer ring face of the split ring 21. By adopting the above solution, a high connection strength between the split ring 21 and the annular disk 24 can be ensured, while ensuring the split ring 21 has a certain degree of elasticity.

It should be understood that, in other embodiments, the length of the portion of the outer ring surface of the split ring 21 fixedly connected to the inner ring face of the annular disk 24 may also be greater than half of the length of the outer ring face of the split ring 21.

In one of the embodiments, a position of a junction of separation and connection between the outer ring surface of the split ring 21 and the inner ring face of the annular disk 24 (approximately a position of point A shown in the figure) is opposite to a position of the opening 213 of the split ring 21.

The split ring 21 is coplanar with a side of the annular disk 24 away from the sewing ring 10. That is, a surface 214 of the split ring 21 away from the sewing ring 10 is coplanar with a surface 243 of the annular disk 24 away from the sewing ring 10, thereby reducing a thickness of the clamping assembly 20 and simplifying the structure. Specifically, a surface of the split ring 21 facing the sewing ring 10 is coplanar with a surface of the annular disk 24 facing the sewing ring 10.

Referring to FIG. 6, in some embodiments, a flange 244 is provided on an outer periphery of a side of the annular disk 24 away from the sewing ring 10, so that a recessed limiting area 35 can be formed between the sewing ring 10 and the flange 244 for a surgical clamp to clamp the ventricular connecting assembly 100, which is convenient for an operator to clamp the ventricular connecting assembly 100 with the surgical clamp during the operation.

In one of the embodiments, the split ring 21 and the annular disk 24 are integrally formed. For example, an end of an open annular plate may be provided with a slot extending along a circumferential direction, and the slot extends from an end of the annular plate to a middle of the annular plate in the circumferential direction, so that the split ring 21 and annular disk 24 are integrally formed, or, may be obtained by metal injection molding, metal casting molding and other methods. In some embodiments, the split ring 21, the first connecting arm 22, the second connecting arm 23, and the annular disk 24 are integrally formed, that is, the entire clamping assembly 20 is integrally formed. At this time, the integrally formed clamping assembly 20 can still be manufactured by the above method.

Referring to FIG. 2 and FIG. 3 again, the locking assembly 40 can adjust the inner diameter of the split ring 21 and maintain the split ring 21 in the clamping state. The locking assembly 40 includes a connecting member 41 and a locking member 42. The connecting member 41 is connected to the second connecting arm 23, and the locking member 42 is rotatably connected to the connecting member 41. In an embodiment, an independent rotating shaft 43 may be provided, which can be movably extend through the locking member 42 and the connecting member 41. Alternatively, the rotating shaft 43 is fixedly connected to the locking member 42, and the connecting member 41 is rotatably connected to the rotating shaft 43. Alternatively, the rotating shaft 43 is rotatably connected to the locking member 42 and fixedly connected to the connecting member 41.

The locking member 42 can rotatably abut against the first connecting arm 22. When the locking member 42 rotates, it can adjust relative positions of the first connecting arm 22 and the second connecting arm 23, so as to adjust the inner diameter of the split ring 21, and the switching of the split ring 21 between the clamping state and the non-clamping state can be achieved.

The locking member 42 is engaged with the second connecting arm 23 to maintain the relative positions of the first connecting arm 22 and the second connecting arm 23, and to keep the distance between the first connecting arm 22 and the second connecting arm 23 unchanged, so as to maintain the split ring 21 in the clamping state, so that the inlet tube of the blood pump is clamped and fixed. By rotating the locking member 42 relative to the first connecting arm 23, the locking member 42 can be engaged with and disengaged from the second connecting arm 23.

In the ventricular connecting assembly 100 according to the embodiment of the present application, the relative positions of the first connecting arm 22 and the second connecting arm 23 are adjusted by rotating the locking member 42, so as to adjust the inner diameter of the split ring 21, so that the split ring 21 can be switched between the clamping state and the non-clamping state to realize the locking and loosening of the inlet tube of the blood pump, and the operation is simple and convenient. By engaging the locking member 42 with the second connecting arm 23, the relative positions of the first connecting arm 22 and the second connecting arm 23 can be maintained, and the split ring 21 is maintained in the clamping state, so that the inlet tube of the blood pump is clamped and fixed without using sutures or fasteners to maintain the split ring 21 in the clamping state, so that the mounting of the blood pump is also relatively simple.

In one of the embodiments, the first connecting arm 22 is a movable arm, and the second connecting arm 23 is a fixed arm. When the locking member 42 rotates, the first connecting arm 22 can move toward or away from the second connecting arm 23. In some other embodiments, both the first connecting arm 22 and the second connecting arm 23 are movable arms, and when the locking member 42 rotates, the first connecting arm 22 and the second connecting arm 23 can move toward or away from each other. Alternatively, the first connecting arm 22 is a fixed arm, the second connecting arm 23 is a movable arm, and when the locking member 42 rotates, the second connecting arm 23 can move toward or away from the first connecting arm 22.

In some embodiments, in order to facilitate the rotation of the locking member 42 to adjust the relative positions of the first connecting arm 22 and the second connecting arm 23, the relative positions of the first connecting arm 22 and the second connecting arm 23 are maintained by engaging the locking member 42 with the second connecting arm 23. The locking member 42 is capable of rotatably abutting against the side of the first connecting arm 22 away from the second connecting arm 23. When the locking member 42 is engaged with the second connecting arm 23, a portion of the locking member 42 that engages with the second connecting arm 23 exerts a force or force component in a direction toward the first connecting arm 23 on the second connecting arm 23. That is, when the locking member 42 is engaged with the second connecting arm 23, the locking member 42 applies a first force or a first component force to the first connecting arm 22 in a direction toward the second connecting arm 22, and a portion of the locking member 42 engaged with the second connecting arm 23 exerts a second force or a second force component to the second connecting arm 23 in a direction toward the first connecting arm 22. Under the action of the first force or the first component force, the second force or the second component force, the relative positions of the first connecting arm 22 and the second connecting arm 23 will be maintained. Thus, the way of engaging the locking member 42 with the second connecting arm 23 may be, for example, that the locking member 42 is engaged with the side of the second connecting arm 23 away from the first connecting arm 22. Alternatively, a cooperating portion is provided on the second connecting arm 23 that cooperates with the locking member 42. The cooperating portion may be a groove whose opening is away from the first connecting arm 22, a surface away from the first connecting arm 22, or a protrusion extending away from the first connecting arm 22.

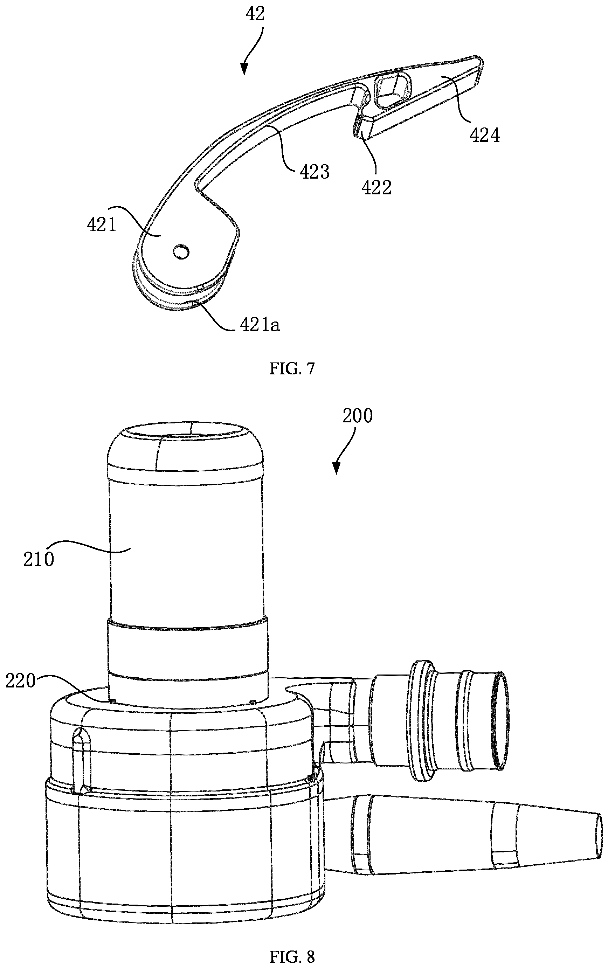

Referring to FIG. 7, in an embodiment, the locking member 42 includes a cam end 421, and the cam end 421 abuts against the side of the first connecting arm 22 away from the second connecting arm 23. The cam end 421 can rotate relative to the first connecting arm 22, so as to adjust the relative positions of the first connecting arm 22 and the second connecting arm 23. During the rotation relative to the first connecting arm 22, the cam end 421 can push the first connecting arm 22 away from or adjacent to the second connecting arm 23, so that the relative positions of the first connecting arm 22 and the second connecting arm 23 are adjusted, and the inner diameter of the split ring 21 is further adjusted, therefore the split ring 21 can be switched between the clamping state and the non-clamping state.

By adopting the above solution, the distance between the first connecting arm 22 and the second connecting arm 23 is adjusted by the rotation of the cam end 421 relative to the first connecting arm 22, which not only can simplify the operation, but also can strengthen the snapping stability between the locking member 42 and the second connecting arm 23, further ensuring that the risk of loosening between the locked locking member 42 and the second connecting arm 23 is reduced.

Referring to FIG. 5 and FIG. 7, in some embodiments, the side of the second connecting arm 23 away from the first connecting arm 22 is provided with an engaging portion 231, and the locking member 42 is provided with a cooperation portion 422 cooperating with the engaging portion 231. The cooperation portion 422 can be engaged with and disengaged from the engaging portion 231 by rotating the locking member 42 relative to the first connecting arm 22. Specifically, the engaging portion 231 is a groove, and the cooperation portion 422 is a protrusion, or the engaging portion 231 is a protrusion, and the cooperation portion 422 is a groove.

In the illustrated embodiment, the locking member 42 further includes a connecting portion 423 having an end fixedly connected to the cam end 421, and a handle portion 424 fixedly connected to an end of the connecting portion 423 away from the cam end 421, and the cooperation portion 422 is provided on the handle portion 424. The cam end 421 is arranged opposite to the cooperation portion 422. Specifically, the connecting portion 423 is shaped substantially as an arc strip.

By adopting the above solution, the locking member 42 can be quickly engaged with the second connecting arm 23, and the locking member 42 can be stably engaged with the second connecting arm 23, so that the inlet tube of the blood pump can be clamped and fixed. The structure is simple and the operation is quick.

In some embodiments, the first connecting arm 22 is provided with a guiding groove 222. One end of the connecting member 41 is fixedly connected to the second connecting arm 23, the other end of the connecting member 41 is rotatably connected to the locking member 42, and a middle portion of the connecting member 41 slidably extends through the guiding groove 222. Such configuration can limit the position of the connecting member 41 and prevent the connecting member 41 from being deflected, thereby preventing the connecting member 41 from being deflected when the locking member 42 rotates relative to the second connecting arm 23, and further ensuring that the first connecting arm 22 and the second connecting arm 23 will not be misaligned when clamping the inlet tube of the blood pump.

Referring to FIG. 3 again, in some embodiments, the connecting member 41 includes a rod portion 411 and a limiting block 412 provided at an end of the rod portion 411. An end of the rod portion 411 away from the limiting block 412 is rotatably connected to the locking member 42, and the limiting block 412 is capable of abutting against the side of the second connecting arm 23 adjacent to the first connecting arm 22, so as to prevent the first connecting arm 22 from continuously moving in a direction toward the second connecting arm 23, and keep the relative positions of the first connecting arm 22 and the second connecting arm 23. The risk of the locking member 42 coming loose from the second connecting arm 23 is reduced. Specifically, the rod portion 411 of the connecting member 41 slidably extends through the guiding groove 222.

Referring to FIG. 7 again, in the illustrated embodiment, the cam end 421 of the locking member 42 is provided with two spaced cam portions 421a. The end of the first connecting arm 22 away from the split ring 21 is formed with a U-shaped portion 224, the U-shaped portion 224 surrounds the guiding groove 222. The two cam portions 421a are capable of rotatably abutting against two arms of the U-shaped portion 224, respectively. The rotating shaft 43 extends through the connecting member 41, and two ends of the rotating shaft 43 are connected to the two cam portions 421a, respectively. The two cam portions 421a are parallel.

Referring to FIG. 1, FIG. 3, FIG. 4, and FIG. 8, the embodiment of a second aspect of the present application provides a ventricular assist system, which includes a blood pump 200 and the above-mentioned ventricular connecting assembly 100.

The blood pump 200 includes an inlet tube 210. The inlet tube 210 extends through the split ring 21, and the split ring 21 is capable of clamping the inlet tube 210 when the split ring 21 is in a clamping state.

In the ventricular assist system of the present application, since the locking member 42 is capable of adjusting the relative positions of the first connecting arm 22 and the second connecting arm 23 when rotating, the inner diameter of the split ring 21 can be adjusted, so that the split ring 21 can be switched between the clamping state and the non-clamping state, the inlet tube 210 of the blood pump 200 can be clamped or loosened, the structure is simple, the operation is convenient, and the mounting of the blood pump 200 is convenient.

In some embodiments, an inner ring wall of the split ring 21 is provided with a positioning groove 215, an outer peripheral surface of the inlet tube 210 is provided with a positioning protrusion 220, and the positioning protrusion 220 is capable of being accommodated in the positioning groove 215 to prevent the inlet tube 210 and the split ring 21 from rotating relative to each other.

By adopting the above solution, when the inlet tube 210 is clamped and fixed by the ventricular connecting assembly 100, the relative rotation between the inlet tube 210 and the ventricular connecting assembly 100 can be avoided.

In some embodiments, a plurality of positioning protrusions 220 are provided, which can improve the ability of the positioning protrusions 220 to limit the relative rotation between the inlet tube 210 and the split ring 21. The plurality of positioning protrusions 220 surround the inlet tube 210 to limit the relative rotation of the inlet tube 210 and the split ring 21 at a plurality of positions, thereby improving the stability thereof.

In the embodiment, the positioning groove 214 is provided on the inner ring wall of the portion of the split ring 21 fixedly connected to the annular disk 24, and the inner ring wall of the portion of the split ring 21 that is separated from the annular disk 24 is further provided with an avoidance groove 216 extending in the circumferential direction of the split ring 21. Part of the positioning protrusions 220 are capable of being accommodated in the positioning groove 215 to limit the relative rotation of the inlet tube 210 and the split ring 21, and the rest part of positioning protrusions 220 are capable of being accommodated in the avoidance groove 216 to prevent the remaining positioning protrusions 220 from interfering with the inner ring wall of the split ring 21.

It should be noted that the ventricular connecting assembly is not limited to the above structure. In some embodiments, the sewing ring 10 can also be omitted, and the clamping assembly 40 can be directly sutured on the biological tissue.

The clamping assembly 20 is not limited to the above-mentioned structure. In some embodiments, the split ring 21 is not arranged in the inner ring of the annular disk 24, and the split ring 21 and the annular disk 24 are arranged coaxially. At this time, the annular disk 24 may be a complete ring structure instead of an open annular structure, or, in some embodiments, the split ring 21 can also be partially accommodated in the inner ring of the annular disk 24, or, in other embodiments, the clamping assembly 20 does not have the annular disk 24, and a portion of the outer peripheral surface of the split ring 21 is bent and extended to form a flange configured to be fixed connected to the sewing ring 10 or biological tissue.

## Claims

1. A ventricular connecting assembly (100), comprising:
a clamping assembly (20) comprising a split ring (21), a first connecting arm (22), and a second connecting arm (23), the split ring (21) comprising a first end (211) and a second end (212) spaced apart from the first end (211), an inner diameter of the split ring (21) being adjustable, so that the split ring (21) has a clamping state and a non-clamping state, and the first connecting arm (22) and the second connecting arm (23) being connected to the first end (211) and the second end (212) of the split ring (21), respectively; and
a locking assembly (40) comprising a connecting member (41) and a locking member (42), the connecting member (41) being connected to the second connecting arm (23), the locking member (42) being rotatably connected to the connecting member (41), the locking member (42) being capable of rotatably abutting against the first connecting arm (22), wherein the locking member (42) is capable of adjusting relative positions of the first connecting arm (22) and the second connecting arm (23) when the locking member (42) rotates, so as to adjust the inner diameter of the split ring (21), and the locking member (42) is engaged with the second connecting arm (23) to maintain the split ring (21) in the clamped state.

2. The ventricular connecting assembly (100) according to claim 1, wherein the locking member (42) comprises a cam end (421), the cam end (421) is capable of rotatably abutting against a side of the first connecting arm (22) away from the second connecting arm (23), and the cam end (421) is capable of adjusting the relative positions of the first connecting arm (22) and the second connecting arm (23) when the cam end (421) rotates relative to the first connecting arm (22).

3. The ventricular connecting assembly (100) according to claim 2, wherein the locking member (42) further comprises a connecting portion (423) and a handle portion (424), an end of the connecting portion (423) is fixedly connected to the cam end (421), the handle portion (424) is fixedly connected to an end of the connecting portion (423) away from the cam end (421), a side of the second connecting arm (23) away from the first connecting arm (22) is provided with an engaging portion (231), the locking member (42) comprises a cooperation portion (422) cooperating with the engaging portion (231), and the cooperation portion (422) is provided on the handle portion (424) and is arranged opposite to the cam end (421).

4. The ventricular connecting assembly (100) according to claim 3, wherein the cam end (421) of the locking member (42) is provided with two spaced cam portions (421), an end of the first connecting arm (22) away from the split ring (21) is formed with a U-shaped portion, and the two cam portions (421) are capable of rotatably abutting against two arms of the U-shaped portion, respectively.

5. The ventricular connecting assembly (100) according to claim 1, wherein a side of the second connecting arm (23) away from the first connecting arm (22) is provided with an engaging portion (231), the locking member (42) is provided with a cooperation portion (422) cooperating with the engaging portion (231), and the cooperation portion (422) is engaged with and disengaged from the engaging portion (231) by rotating the locking member (42) relative to the first connecting arm (22).

6. The ventricular connecting assembly (100) according to claim 1, wherein the connecting member (41) comprises a rod portion (411) and a limiting block (412) provided at an end of the rod portion (411), an end of the rod portion (411) away from the limiting block (412) is rotatably connected to the locking member (42), and the limiting block (412) is capable of abutting against a side of the second connecting arm (23) adjacent to the first connecting arm (22);
and/or wherein the first connecting arm (22) is provided with a guiding groove (222), one end of the connecting member (41) is fixedly connected to the second connecting arm (23), the other end of the connecting member (41) is rotatably connected to the locking member (42), and a middle portion of the connecting member (41) slidably extends through the guiding groove (222).

7. The ventricular connecting assembly (100) according to any one of claims 1 to 6, wherein the clamping assembly (20) further comprises an annular disk (24) having an opening (242), the split ring (21) is provided in an inner ring of the annular disk (24), a position of an opening (213) of the split ring (21) corresponds to a position of the opening (242) of the annular disk (24), an outer ring surface of the split ring (21) adjacent to the second end (212) is fixedly connected to an inner ring surface of the annular disk (24), and an outer ring surface of the split ring (21) adjacent to the first end (211) is separated from the annular disk (24).

8. The ventricular connecting assembly (100) according to claim 7, wherein a length of a portion of the outer ring surface of the split ring (21) fixedly connected to the inner ring face of the annular disk (24) is less than or equal to half of a length of the outer ring face of the split ring (21);
and/or wherein a position of a junction of separation and connection between the outer ring surface of the split ring (21) and the inner ring face of the annular disk (24) is opposite to a position of the opening (213) of the split ring (21).

9. The ventricular connecting assembly (100) according to claim 7, further comprising a sewing ring (10) fixedly connected to the annular disk (24), wherein the split ring (21) is coplanar with a side of the annular disk (24) away from the sewing ring (10), and/or a flange (244) is provided on an outer periphery of a side of the annular disk (24) away from the sewing ring (10).

10. A ventricular assist system, comprising:
a blood pump comprising an inlet tube; and
the ventricular connecting assembly (100) according to any one of claims 1 to 9, wherein the inlet tube extends through the split ring (21), and the split ring (21) is capable of clamping the inlet tube when the split ring (21) is in the clamping state.

11. The ventricular assist system according to claim 10, wherein an inner ring wall of the split ring (21) is provided with a positioning groove (215), an outer peripheral surface of the inlet tube is provided with a positioning protrusion (220), and the positioning protrusion (220) is capable of being accommodated in the positioning groove (215).

12. A ventricular assist system, comprising a blood pump and a ventricular connecting assembly (100), the blood pump comprising an inlet tube, the ventricular connecting assembly (100) comprising:
a sewing ring (10);
a clamping assembly (20) comprising a split ring (21), a first connecting arm (22), and a second connecting arm (23), the split ring (21) comprising a first end (211) and a second end (212) spaced apart from the first end (211), an inner diameter of the split ring (21) being adjustable, so that the split ring (21) has a clamping state and a non-clamping state, and the first connecting arm (22) and the second connecting arm (23) being connected to the first end (211) and the second end (212) of the split ring (21), respectively, the annular disk (24) being annular with an opening (242), and the annular disk (24) being connected with the sewing ring (10), wherein the split ring (21) is provided in an inner ring of the annular disk (24), a position of the opening (213) of the split ring (21) corresponds to a position of the opening (242) of the annular disk (24), an outer ring surface of the split ring (21) adjacent to the second end (212) is fixedly connected to the inner ring surface of the annular disk (24), and an outer ring surface of the split ring (21) adjacent to the first end (211) is separated from the annular disk (24);
a locking assembly (40) comprising a connecting member (41) and a locking member (42), the connecting member (41) being connected to the second connecting arm (23), the locking member (42) being rotatably connected to the connecting member (41), and the locking member (42) being capable of rotatably abutting against the first connecting arm (22), wherein the locking member (42) is capable of adjusting relative positions of the first connecting arm (22) and the second connecting arm (23) when the locking member (42) rotates, so as to adjust the inner diameter of the split ring (21), and the locking member (42) is engaged with the second connecting arm (23) to maintain the split ring (21) in the clamped state;
wherein the inlet tube extends through the split ring (21), and the split ring (21) is capable of clamping the inlet tube when the split ring (21) is in the clamping state.

13. The ventricular assist system according to claim 12, wherein an inner ring wall of the split ring (21) is provided with a positioning groove (215), an outer peripheral surface of the inlet tube is provided with a positioning protrusion (220), and the positioning protrusion (220) is capable of being accommodated in the positioning groove (215).

14. The ventricular assist system according to claim 13, wherein a plurality of positioning protrusions (220) are provided, and the plurality of positioning protrusions (220) surround the inlet tube.

15. The ventricular assist system according to claim 14, wherein the positioning groove (215) is provided on the inner ring wall of a portion of the split ring (21) fixedly connected to the annular disk (24), the inner ring wall of a portion of the split ring (21) that is separated from the annular disk (24) is further provided with an avoidance groove (216) extending in a circumferential direction of the split ring (21), part of the positioning protrusions (220) are capable of being accommodated in the positioning groove (215), and the rest part of positioning protrusions (220) are capable of being accommodated in the avoidance groove (216).

## Patentansprüche

1. Ventrikuläre Verbindungsbaugruppe (100), umfassend:
eine Klemmbaugruppe (20), die einen Spaltring (21), einen ersten Verbindungsarm (22) und einen zweiten Verbindungsarm (23) umfasst, wobei der Spaltring (21) ein erstes Ende (211) und ein von dem ersten Ende (211) beabstandetes zweites Ende (212) umfasst, wobei ein Innendurchmesser des Spaltrings (21) verstellbar ist, so dass der Spaltring (21) einen Klemmzustand und einen nicht klemmenden Zustand aufweist, und wobei der erste Verbindungsarm (22) und der zweite Verbindungsarm (23) mit dem ersten Ende (211) bzw. dem zweiten Ende (212) des Spaltrings (21) verbunden sind, und
eine Verriegelungsbaugruppe (40), die ein Verbindungsglied (41) und ein Verriegelungsglied (42) umfasst, wobei das Verbindungsglied (41) mit dem zweiten Verbindungsarm (23) verbunden ist, wobei das Verriegelungsglied (42) drehbar mit dem Verbindungsglied (41) verbunden ist, wobei das Verriegelungsglied (42) drehbar an dem ersten Verbindungsarm (22) anliegen kann, wobei das Verriegelungsglied (42) relative Positionen des ersten Verbindungsarms (22) und des zweiten Verbindungsarms (23) verstellen kann, wenn sich das Verriegelungsglied (42) dreht, um den Innendurchmesser des Spaltrings (21) zu verstellen, und das Verriegelungsglied (42) mit dem zweiten Verbindungsarm (23) in Eingriff ist, um den Spaltring (21) in dem geklemmten Zustand zu halten.

2. Ventrikuläre Verbindungsbaugruppe (100) nach Anspruch 1, wobei das Verriegelungsglied (42) ein Nockenende (421) umfasst, wobei das Nockenende (421) drehbar an einer Seite des ersten Verbindungsarms (22) weg von dem zweiten Verbindungsarm (23) anliegen kann und das Nockenende (421) die relativen Positionen des ersten Verbindungsarms (22) und des zweiten Verbindungsarms (23) zu verstellen, wenn sich das Nockenende (421) relativ zu dem ersten Verbindungsarm (22) dreht.

3. Ventrikuläre Verbindungsbaugruppe (100) nach Anspruch 2, wobei das Verriegelungsglied (42) ferner einen Verbindungsabschnitt (423) und einen Griffabschnitt (424) umfasst, wobei ein Ende des Verbindungsabschnitts (423) fest mit dem Nockenende (421) verbunden ist, der Griffabschnitt (424) fest mit einem Ende des Verbindungsabschnitts (423) von dem Nockenende (421) weg verbunden ist, eine von dem ersten Verbindungsarm (22) weg liegende Seite des zweiten Verbindungsarms (23) mit einem Eingriffsabschnitt (231) versehen ist, das Verriegelungsglied (42) einen mit dem Eingriffsabschnitt (231) zusammenwirkenden Zusammenwirkungsabschnitt (422) umfasst und der Mitwirkungsabschnitt (422) an dem Griffabschnitt (424) vorgesehen und gegenüber dem Nockenende (421) angeordnet ist.

4. Ventrikuläre Verbindungsbaugruppe (100) nach Anspruch 3, wobei das Nockenende (421) des Verriegelungsglieds (42) mit zwei beabstandeten Nockenabschnitten (421) versehen ist, ein von dem Spaltring (21) weg liegendes Ende des ersten Verbindungsarms (22) mit einem U-förmigen Abschnitt ausgebildet ist und die beiden Nockenabschnitte (421) jeweils drehbar an zwei Armen des U-förmigen Abschnitts anliegen können.

5. Ventrikuläre Verbindungsbaugruppe (100) nach Anspruch 1, wobei eine von dem ersten Verbindungsarm (22) weg liegende Seite des zweiten Verbindungsarms (23) mit einem Eingriffsabschnitt (231) versehen ist, das Verriegelungsglied (42) mit einem mit dem Eingriffsabschnitt (231) zusammenwirkenden Zusammenwirkungsabschnitt (422) versehen ist und der Zusammenwirkungsabschnitt (422) durch Drehen des Verriegelungsglieds (42) bezüglich des ersten Verbindungsarms (22) mit dem Eingriffsabschnitt (231) in Eingriff gebracht und daraus ausgerückt wird.

6. Ventrikuläre Verbindungsbaugruppe (100) nach Anspruch 1, wobei das Verbindungsglied (41) einen Stangenabschnitt (411) und einen Begrenzungsblock (412) umfasst, der an einem Ende des Stangenabschnitts (411) vorgesehen ist, ein von dem Begrenzungsblock (412) weg liegendes Ende des Stangenabschnitts (411) drehbar mit dem Verriegelungsglied (42) verbunden ist und der Begrenzungsblock (412) an einer Seite des zweiten Verbindungsarms (23) neben dem ersten Verbindungsarm (22) anliegen kann,
und/oder wobei der erste Verbindungsarm (22) mit einer Führungsnut (222) versehen ist, ein Ende des Verbindungsglieds (41) fest mit dem zweiten Verbindungsarm (23) verbunden ist, das andere Ende des Verbindungsglieds (41) drehbar mit dem Verriegelungsglied (42) verbunden ist und ein Mittelabschnitt des Verbindungsglieds (41) sich gleitend durch die Führungsnut (222) erstreckt.

7. Ventrikuläre Verbindungsbaugruppe (100) nach einem der Ansprüche 1 bis 6, wobei die Klemmbaugruppe (20) ferner eine Ringscheibe (24) mit einer Öffnung (242) aufweist, wobei der Spaltring (21) in einem inneren Ring der Ringscheibe (24) vorgesehen ist, wobei eine Position einer Öffnung (213) des Spaltrings (21) einer Position der Öffnung (242) der Ringscheibe (24) entspricht, wobei eine äußere Ringfläche des Spaltrings (21) neben dem zweiten Ende (212) fest mit einer inneren Ringfläche der Ringscheibe (24) verbunden ist und eine äußere Ringfläche des Spaltrings (21) neben dem ersten Ende (211) von der Ringscheibe (24) getrennt ist.

8. Ventrikuläre Verbindungsbaugruppe (100) nach Anspruch 7, wobei eine Länge eines Abschnitts der äußeren Ringfläche des Spaltrings (21), der fest mit der inneren Ringfläche der Ringscheibe (24) verbunden ist, kleiner oder gleich einer halben Länge der äußeren Ringfläche des Spaltrings (21) ist
und/oder wobei eine Position eines Übergangs von Trennung und Verbindung zwischen der äußeren Ringfläche des Spaltrings (21) und der inneren Ringfläche der Ringscheibe (24) einer Position der Öffnung (213) des Spaltrings (21) gegenüberliegt.

9. Ventrikuläre Verbindungsbaugruppe (100) nach Anspruch 7, ferner umfassend einen fest mit der Ringscheibe (24) verbundenen Nähring (10), wobei der Spaltring (21) mit einer von dem Nähring (10) weg liegenden Seite der Ringscheibe (24) koplanar ist und/oder ein Flansch (244) an einem Außenumfang einer von dem Nähring (10) weg liegenden Seite der Ringscheibe (24) vorgesehen ist.

10. Ventrikuläres Unterstützungssystem, umfassend:
eine Blutpumpe, die eine Einlassröhre umfasst, und
die ventrikuläre Verbindungsbaugruppe (100) nach einem der Ansprüche 1 bis 9, wobei sich die Einlassröhre durch den Spaltring (21) erstreckt und der Spaltring (21) die Einlassröhre klemmen kann, wenn der Spaltring (21) im Klemmzustand ist.

11. Ventrikuläres Unterstützungssystem nach Anspruch 10, wobei eine innere Ringwand des Spaltrings (21) mit einer Positionierungsnut (215) versehen ist, eine äußere Umfangsfläche der Einlassröhre mit einem Positionierungsvorsprung (220) versehen ist und der Positionierungsvorsprung (220) in der Positionierungsnut (215) aufgenommen werden kann.

12. Ventrikuläres Unterstützungssystem, umfassend eine Blutpumpe und eine ventrikuläre Verbindungsbaugruppe (100), wobei die Blutpumpe eine Einlassröhre umfasst, wobei die ventrikuläre Verbindungsbaugruppe (100) Folgendes umfasst:
einen Nähring (10),
eine Klemmbaugruppe (20), die einen Spaltring (21), einen ersten Verbindungsarm (22) und einen zweiten Verbindungsarm (23) umfasst, wobei der Spaltring (21) ein erstes Ende (211) und ein von dem ersten Ende (211) beabstandetes zweites Ende (212) umfasst, wobei ein Innendurchmesser des Spaltrings (21) verstellbar ist, so dass der Spaltring (21) einen Klemmzustand und einen nicht klemmenden Zustand aufweist und wobei der erste Verbindungsarm (22) und der zweite Verbindungsarm (23) mit dem ersten Ende (211) bzw. dem zweiten Ende (212) des Spaltrings (21) verbunden sind, wobei die Ringscheibe (24) ringförmig mit einer Öffnung (242) ist und wobei die Ringscheibe (24) mit dem Nähring (10) verbunden ist, wobei der Spaltring (21) in einem inneren Ring der Ringscheibe (24) vorgesehen ist, wobei eine Position der Öffnung (213) des Spaltrings (21) einer Position der Öffnung (242) der Ringscheibe (24) entspricht, wobei eine äußere Ringfläche des Spaltrings (21) neben dem zweiten Ende (212) fest mit der inneren Ringfläche der Ringscheibe (24) verbunden ist und eine äußere Ringfläche des Spaltrings (21) neben dem ersten Ende (211) von der Ringscheibe (24) getrennt ist,
eine Verriegelungsbaugruppe (40), die ein Verbindungsglied (41) und ein Verriegelungsglied (42) umfasst, wobei das Verbindungsglied (41) mit dem zweiten Verbindungsarm (23) verbunden ist, wobei das Verriegelungsglied (42) drehbar mit dem Verbindungsglied (41) verbunden ist, wobei das Verriegelungsglied (42) drehbar an dem ersten Verbindungsarm (22) anliegen kann, wobei das Verriegelungsglied (42) relative Positionen des ersten Verbindungsarms (22) und des zweiten Verbindungsarms (23) verstellen kann, wenn sich das Verriegelungsglied (42) dreht, um den Innendurchmesser des Spaltrings (21) zu verstellen, und das Verriegelungsglied (42) mit dem zweiten Verbindungsarm (23) in Eingriff ist, um den Spaltring (21) in dem geklemmten Zustand zu halten,
wobei sich die Einlassröhre durch den Spaltring (21) erstreckt und der Spaltring (21) die Einlassröhre klemmen kann, wenn sich der Spaltring (21) im Klemmzustand befindet.

13. Ventrikuläres Unterstützungssystem nach Anspruch 12, wobei eine innere Ringwand des Spaltrings (21) mit einer Positionierungsnut (215) versehen ist, eine äußere Umfangsfläche der Einlassröhre mit einem Positionierungsvorsprung (220) versehen ist und der Positionierungsvorsprung (220) in der Positionierungsnut (215) aufgenommen werden kann.

14. Ventrikuläres Unterstützungssystem nach Anspruch 13, wobei eine Vielzahl von Positionierungsvorsprüngen (220) vorgesehen sind und die Vielzahl von Positionierungsvorsprüngen (220) die Einlassröhre umgeben.

15. Ventrikuläres Unterstützungssystem nach Anspruch 14, wobei die Positionierungsnut (215) an der inneren Ringwand eines Abschnitts des Spaltrings (21) vorgesehen ist, der fest mit der Ringscheibe (24) verbunden ist, wobei die innere Ringwand eines Abschnitts des Spaltrings (21), der von der Ringscheibe (24) getrennt ist, ferner mit einer Ausweichnut (216) versehen ist, die sich in einer Umfangsrichtung des Spaltrings (21) erstreckt, wobei ein Teil der Positionierungsvorsprünge (220) in der Positionierungsnut (215) aufgenommen werden können und der restliche Teil der Positionierungsvorsprünge (220) in der Ausweichnut (216) aufgenommen werden können.

## Revendications

1. Ensemble de liaison ventriculaire (100), comprenant :
un ensemble de serrage (20) comprenant une bague fendue (21), un premier bras de liaison (22) et un second bras de liaison (23), la bague fendue (21) comprenant une première extrémité (211) et une seconde extrémité (212) espacée de la première extrémité (211), un diamètre intérieur de la bague fendue (21) étant réglable, de sorte que la bague fendue (21) présente un état de serrage et un état de non-serrage, et le premier bras de liaison (22) et le second bras de liaison (23) étant reliés respectivement à la première extrémité (211) et à la seconde extrémité (212) de la bague fendue (21) ; et
un ensemble de verrouillage (40) comprenant un élément de liaison (41) et un élément de verrouillage (42), l'élément de liaison (41) étant relié au second bras de liaison (23), l'élément de verrouillage (42) étant relié de manière rotative à l'élément de liaison (41), l'élément de verrouillage (42) étant susceptible de buter de manière rotative contre le premier bras de liaison (22), l'élément de verrouillage (42) étant susceptible de régler des positions relatives du premier bras de liaison (22) et du second bras de liaison (23) lorsque l'élément de verrouillage (42) tourne, de manière à régler le diamètre intérieur de la bague fendue (21), et l'élément de verrouillage (42) étant en prise avec le second bras de liaison (23) pour maintenir la bague fendue (21) dans l'état serré.

2. Ensemble de liaison ventriculaire (100) selon la revendication 1, dans lequel l'élément de verrouillage (42) comprend une extrémité de came (421), l'extrémité de came (421) est susceptible de venir en butée rotative contre un côté du premier bras de liaison (22) éloigné du second bras de liaison (23), et l'extrémité de came (421) est susceptible de régler les positions relatives du premier bras de liaison (22) et du second bras de liaison (23) lorsque l'extrémité de came (421) tourne par rapport au premier bras de liaison (22).

3. Ensemble de liaison ventriculaire (100) selon la revendication 2, dans lequel l'élément de verrouillage (42) comprend en outre une partie de liaison (423) et une partie poignée (424), une extrémité de la partie de liaison (423) est reliée à demeure à l'extrémité de came (421), la partie poignée (424) est reliée à demeure à une extrémité de la partie de liaison (423) éloignée de l'extrémité de came (421), un côté du second bras de liaison (23) éloigné du premier bras de liaison (22) est pourvu d'une partie d'entrée en prise (231), l'élément de verrouillage (42) comprend une partie de coopération (422) coopérant avec la partie d'entrée en prise (231), et la partie de coopération (422) est disposée sur la partie poignée (424) et est disposée à l'opposé de l'extrémité de came (421).

4. Ensemble de liaison ventriculaire (100) selon la revendication 3, dans lequel l'extrémité de came (421) de l'élément de verrouillage (42) est pourvue de deux parties de came espacées (421), une extrémité du premier bras de liaison (22) éloignée de la bague fendue (21) est formée avec une partie en forme de U, et les deux parties de came (421) sont susceptibles de buter de manière rotative contre deux bras de la partie en forme de U, respectivement.

5. Ensemble de liaison ventriculaire (100) selon la revendication 1, dans lequel un côté du second bras de liaison (23) éloigné du premier bras de liaison (22) est pourvu d'une partie d'entrée en prise (231), l'élément de verrouillage (42) est pourvu d'une partie de coopération (422) coopérant avec la partie d'entrée en prise (231), et la partie de coopération (422) est en prise avec la partie d'entrée en prise (231) et libérée de celle-ci en faisant tourner l'élément de verrouillage (42) par rapport au premier bras de liaison (22).

6. Ensemble de liaison ventriculaire (100) selon la revendication 1, dans lequel l'élément de liaison (41) comprend une partie tige (411) et un bloc de limitation (412) disposé à une extrémité de la partie tige (411), une extrémité de la partie tige (411) éloignée du bloc de limitation (412) étant reliée de manière rotative à l'élément de verrouillage (42), et le bloc de limitation (412) étant susceptible de buter contre un côté du second bras de liaison (23) adjacent au premier bras de liaison (22) ;
et/ou dans lequel le premier bras de liaison (22) est pourvu d'une rainure de guidage (222), une extrémité de l'élément de liaison (41) est reliée à demeure au second bras de liaison (23), l'autre extrémité de l'élément de liaison (41) est reliée de manière rotative à l'élément de verrouillage (42), et une partie médiane de l'élément de liaison (41) s'étend de manière coulissante à travers la rainure de guidage (222).

7. Ensemble de liaison ventriculaire (100) selon l'une quelconque des revendications 1 à 6, dans lequel l'ensemble de serrage (20) comprend en outre un disque annulaire (24) ayant une ouverture (242), la bague fendue (21) est disposée dans une bague intérieure du disque annulaire (24), une position d'une ouverture (213) de la bague fendue (21) correspond à une position de l'ouverture (242) du disque annulaire (24), une surface annulaire extérieure de la bague fendue (21) adjacente à la seconde extrémité (212) est reliée à demeure à une surface annulaire intérieure du disque annulaire (24), et une surface annulaire extérieure de la bague fendue (21) adjacente à la première extrémité (211) est séparée du disque annulaire (24).

8. Ensemble de liaison ventriculaire (100) selon la revendication 7, dans lequel la longueur d'une partie de la surface annulaire extérieure de la bague fendue (21) reliée à demeure à la face annulaire intérieure du disque annulaire (24) est inférieure ou égale à la moitié de la longueur de la face annulaire extérieure de la bague fendue (21) ;
et/ou dans lequel une position d'une jonction de séparation et de liaison entre la surface annulaire extérieure de la bague fendue (21) et la face annulaire intérieure du disque annulaire (24) est opposée à une position de l'ouverture (213) de la bague fendue (21).

9. Ensemble de liaison ventriculaire (100) selon la revendication 7, comprenant en outre un anneau à coudre (10) relié à demeure au disque annulaire (24), dans lequel la bague fendue (21) est coplanaire avec un côté du disque annulaire (24) éloigné de l'anneau à coudre (10), et/ou une bride (244) est disposée sur une périphérie extérieure d'un côté du disque annulaire (24) éloigné de l'anneau à coudre (10).

10. Système d'assistance ventriculaire, comprenant :
une pompe à sang comprenant un tube d'entrée ; et
l'ensemble de liaison ventriculaire (100) selon l'une quelconque des revendications 1 à 9, dans lequel le tube d'entrée s'étend à travers la bague fendue (21), et la bague fendue (21) est susceptible de serrer le tube d'entrée lorsque la bague fendue (21) est dans l'état de serrage.

11. Système d'assistance ventriculaire selon la revendication 10, dans lequel une paroi annulaire intérieure de la bague fendue (21) est pourvue d'une rainure de positionnement (215), une surface périphérique extérieure du tube d'entrée est pourvue d'une protubérance de positionnement (220), et la protubérance de positionnement (220) est susceptible d'être logée dans la rainure de positionnement (215).

12. Système d'assistance ventriculaire, comprenant une pompe à sang et un ensemble de liaison ventriculaire (100), la pompe à sang comprenant un tube d'entrée, l'ensemble de liaison ventriculaire (100) comprenant :
un anneau à coudre (10) ;
un ensemble de serrage (20) comprenant une bague fendue (21), un premier bras de liaison (22) et un second bras de liaison (23), la bague fendue (21) comprenant une première extrémité (211) et une seconde extrémité (212) espacée de la première extrémité (211), un diamètre intérieur de la bague fendue (21) étant réglable, de sorte que la bague fendue (21) présente un état de serrage et un état de non-serrage, et le premier bras de liaison (22) et le second bras de liaison (23) étant reliés à la première extrémité (211) et à la seconde extrémité (212) de la bague fendue (21), respectivement, le disque annulaire (24) étant annulaire avec une ouverture (242), et le disque annulaire (24) étant relié à l'anneau à coudre (10), dans lequel la bague fendue (21) est disposée dans une bague intérieure du disque annulaire (24), une position de l'ouverture (213) de la bague fendue (21) correspond à une position de l'ouverture (242) du disque annulaire (24), une surface annulaire extérieure de la bague fendue (21) adjacente à la seconde extrémité (212) est reliée à demeure à la surface annulaire intérieure du disque annulaire (24), et une surface annulaire extérieure de la bague fendue (21) adjacente à la première extrémité (211) est séparée du disque annulaire (24) ;
un ensemble de verrouillage (40) comprenant un élément de liaison (41) et un élément de verrouillage (42), l'élément de liaison (41) étant relié au second bras de liaison (23), l'élément de verrouillage (42) étant relié de manière rotative à l'élément de liaison (41), et l'élément de verrouillage (42) étant susceptible de buter de manière rotative contre le premier bras de liaison (22), l'élément de verrouillage (42) étant susceptible de régler des positions relatives du premier bras de liaison (22) et du second bras de liaison (23) lorsque l'élément de verrouillage (42) tourne, de manière à régler le diamètre intérieur de la bague fendue (21), et l'élément de verrouillage (42) étant en prise avec le second bras de liaison (23) pour maintenir la bague fendue (21) dans l'état serré ;
dans lequel le tube d'entrée s'étend à travers la bague fendue (21), et la bague fendue (21) est susceptible de serrer le tube d'entrée lorsque la bague fendue (21) est dans l'état de serrage.

13. Système d'assistance ventriculaire selon la revendication 12, dans lequel une paroi annulaire intérieure de la bague fendue (21) est pourvue d'une rainure de positionnement (215), une surface périphérique extérieure du tube d'entrée est pourvue d'une protubérance de positionnement (220), et la protubérance de positionnement (220) est susceptible d'être logée dans la rainure de positionnement (215).

14. Système d'assistance ventriculaire selon la revendication 13, dans lequel une pluralité de protubérances de positionnement (220) est disposée, et la pluralité de protubérances de positionnement (220) entoure le tube d'entrée.

15. Système d'assistance ventriculaire selon la revendication 14, dans lequel la rainure de positionnement (215) est disposée sur la paroi annulaire intérieure d'une partie de la bague fendue (21) reliée à demeure au disque annulaire (24), la paroi annulaire intérieure d'une partie de la bague fendue (21) qui est séparée du disque annulaire (24) est en outre pourvue d'une rainure d'évitement (216) s'étendant dans une direction circonférentielle de la bague fendue (21), une partie des protubérances de positionnement (220) peut être logée dans la rainure de positionnement (215), et la partie restante des protubérances de positionnement (220) peut être logée dans la rainure d'évitement (216).
